# FASCICULE DE BREVET EUROPEEN

(11) **EP 2 771 282 B1**
(45) Date de publication et mention de la délivrance du brevet: **19.09.2018**
(21) Numéro de dépôt: 12806568.7
(22) Date de dépôt: 26.11.2012
(51) Int. Cl.: C02F 1/32, C02F 1/72, A61L 2/14, A61L 2/10, A61L 2/08, B01D 53/00, B01D 53/32, B01D 53/88

(54) **PROCEDE DE TRAITEMENT D'EFFLUENTS DANS UN LIT DE MICROBILLES PAR PLASMA FROID ET PHOTOCATALYSE**
VERFAHREN ZUR BEHANDLUNG VON ABGASEN IN EINEM BETT AUS MIKROKUGELN DURCH KALTPLASMA UND FOTOKATALYSE
PROCESS FOR TREATING EFFLUENTS IN A BED OF MICROBEADS BY COLD PLASMA AND PHOTOCATALYSIS

(30) Priorité: 01.12.2011 FR 1161056
(43) Date de publication de la demande: 03.09.2014
(73) Titulaire: Beewair, 71850 Charnay-les-Macon (FR)
(72) Inventeur: DEVEAU, Pierre-Alexandre, 26120 Peyrus (FR); PARZY, Didier, 69440 Mornant (FR)
(74) Mandataire: Thibault, Jean-Marc
(86) Numéro de dépôt international: PCT/FR2012/052718
(87) Numéro de publication internationale: WO 2013/079858

(56) Documents cités:
- WO-A1-2006/116828
- US-A1- 2005 118 079
- US-A1- 2006 127 288
- BAN J Y ET AL: "Highly concentrated toluene decomposition on the dielectric barrier discharge (DBD) plasma-photocatalytic hybrid system with Mn-Ti-incorporated mesoporous silicate photocatalyst (Mn-Ti-MPS)", APPLIED SURFACE SCIENCE, ELSEVIER, AMSTERDAM, NL, vol. 253, no. 2, 15 novembre 2006 (2006-11-15), pages 535-542, XP024893621, ISSN: 0169-4332, DOI: 10.1016/J.APSUSC.2005.12.103 [extrait le 2006-11-15] cité dans la demande
- MOREAU M ET AL: "Non-thermal plasma technologies: New tools for bio-decontamination", BIOTECHNOLOGY ADVANCES, ELSEVIER PUBLISHING, BARKING, GB, vol. 26, no. 6, 1 novembre 2008 (2008-11-01), pages 610-617, XP027181989, ISSN: 0734-9750 [extrait le 2008-08-16]

## Description

La présente invention concerne le traitement des effluents gazeux ou aqueux contenant des polluants chimiques, des micro-organismes et/ou des particules, par les techniques combinées de photocatalyse et de plasma froid.

La présente invention vise à traiter les effluents contenant des polluants environnementaux tels que par exemple les particules, les micro-organismes type virus, bactéries, moisissures et algues et les polluants chimiques type COV, SCOV, BTEX, HAP (C10-C25), HAAA, NOx, SOx, H2S, CO, 03, les composés halogénés, les perturbateurs endocriniens et l'ensemble des molécules olfactives.

L'objet de l'invention trouve des applications particulièrement avantageuses mais non exclusivement par exemple dans les milieux industriels chimiques et pétrochimiques, les milieux médicaux et hospitaliers, les lignes de formulation agro-alimentaires, les sites de production et d'élevage alimentaire type avicoles, horticoles, arboricoles et viticoles, les stockages froids de denrées alimentaires périssables type fruits, légumes, poissons, viandes, fromages, pâtisseries, les usines de traitement des eaux, les micro-stations, les cuves de récupération d'eau de pluie, les secteurs tertiaires, privés et collectifs accueillant du public et les habitations, etc..

Le principe du plasma froid généré par une décharge à barrière diélectrique est de former des espèces chimiques excitées type radicaux, anions et cations. Cette génération de plasma gazeux permet d'attaquer les liaisons chimiques des polluants contenus dans un effluent gazeux ou aqueux. Dans la pratique, un plasma froid est obtenu à pression atmosphérique par l'application d'une haute tension entre deux électrodes symétriques séparées par un diélectrique tel une lame d'air. Sous l'effet d'un arc électrique, la lame d'air s'ionise. L'arc électrique ionise les composants contenus dans l'environnement et forme des anions, des cations, des radicaux minoritaires et des espèces excitées. Ces composés dégradent les composés chimiques présents.

Dans l'état de la technique, il est connu d'utiliser le plasma froid généré par une décharge à barrière diélectrique pour la destruction des molécules chimiques et des composés organiques volatils (COV). Selon cette technique, la formation de plasma dans un effluent contenant de l'oxygène à pression atmosphérique permet la formation d'O3. Selon les concentrations, 03 possède un effet germicide sur les micro-organismes.

La photocatalyse est un procédé chimique d'oxydation-réduction mettant en jeu un agent photocatalyseur apte à détruire les différents polluants organiques présents dans l'air ou dans l'eau et ce par une réaction provoquée par l'excitation photonique UV. La photocatalyse assure la formation de radicaux (02 et OH) à la suite de l'irradiation UV avec une longueur d'onde inférieure à 380 nm, d'un semi-conducteur type oxyde-métallique (M-Ox). Lors de l'irradiation UV, le cortège électronique du semi-conducteur de type M-Ox est modifié par le franchissement du GAP électronique par un ou plusieurs électrons. Au contact de l'air et de l'eau, ces électrons forment à la surface du semi-conducteur de type M-Ox des radicaux type radicaux hydroxyles et oxygènes radicalaires. Ces radicaux qui ont des cinétiques réactionnelles très grandes attaquent les composants organiques, chimiques, adsorbées sur les sites activés de M-Ox par les UV et les dégradent en cassant leurs liaisons chimiques. Il peut ainsi être obtenu des dégradations totales ou partielles des composés en CO2, H20, N2, etc.

L'utilisation simple de la photocatalyse sous irradiation UV pour la dégradation des composés chimiques contenus dans des effluents gazeux est décrite par exemple dans la demande de brevet WO 00/72945. Par ailleurs, les deux techniques photocatalyse et plasma froid ont déjà été décrites en combinaison.

Par exemple, la demande de brevet WO 2007/051912 décrit un procédé de traitement des effluents gazeux couplant de manière simultanée le plasma froid de décharge à barrière diélectrique et la photocatalyse avec une source UV extérieure à un réacteur de type léchant. Les parois du diélectrique du dispositif de décharge à barrière diélectrique sont revêtues comme agent photocatalyseur, de TiO2. La décharge de plasma est effectuée au sein du photocatalyseur activé sous irradiation UV. La technique décrite dans ce document présente l'inconvénient que les espèces réactives créées lors de la décharge dans le TiO2 sont majoritairement des anions et des cations dont une fraction est consommée pour générer des radicaux à partir de O2-, O3- et OH-. Cette technique limite la formation d'ozone lors de la décharge mais n'empêche pas sa formation en sortie de réacteur ainsi que la formation de polluants secondaires. Par ailleurs, la géométrie du réacteur ne permet pas d'avoir des temps de minéralisation suffisamment long pour radicaliser l'ensemble des espèces ioniques engendrées par la décharge.

Le brevet US 2002/0168305 décrit également un système de traitement d'air permettant la décontamination des micro-organismes de type virus et bactéries. Ce système de traitement comporte un réacteur annulaire au centre duquel est placée une lampe à décharge permettant la ionisation de l'air et la formation d'ozone. Un diélectrique poreux, compris entre deux manchons en maille métallique, compose les parois du réacteur. Une décharge électrique est réalisée dans cette partie pour permettre la formation d'espèces ioniques et radicalaires ainsi que la consommation de l'ozone formée dans la chambre centrale.

La purification s'effectue en trois étapes :
- irradiation UV et ionisation dans le coeur du réacteur avec génération d'espèces ionique et d'ozone,
- décharge électrique et photocatalyse au sein d'un diélectrique poreux : formation d'ions et d'ozone et formation de radicaux et destruction de l'ozone sur l'ensemble du diélectrique poreux,
- filtration à la sortie.

Cette technique présente les mêmes inconvénients que la demande de brevet WO 2007/051912. La réaction photocatalytique donne des réactions d'oxydo-réduction homolytique générant des radicaux alors que le procédé de décharge électrique donne des réactions hétérolytiques avec des ions. Aussi, la réaction de photocatalyse est plus rapide et donc prédominante, ce qui limite la formation d'un gradient hétérogène d'espèces réactives ions, ozone6 et radicaux au sein du réacteur. La capacité oxydante n'évolue pas et ne permet pas de minéraliser de manière performante et totale, les polluants chimiques et les contaminants micro-organiques.

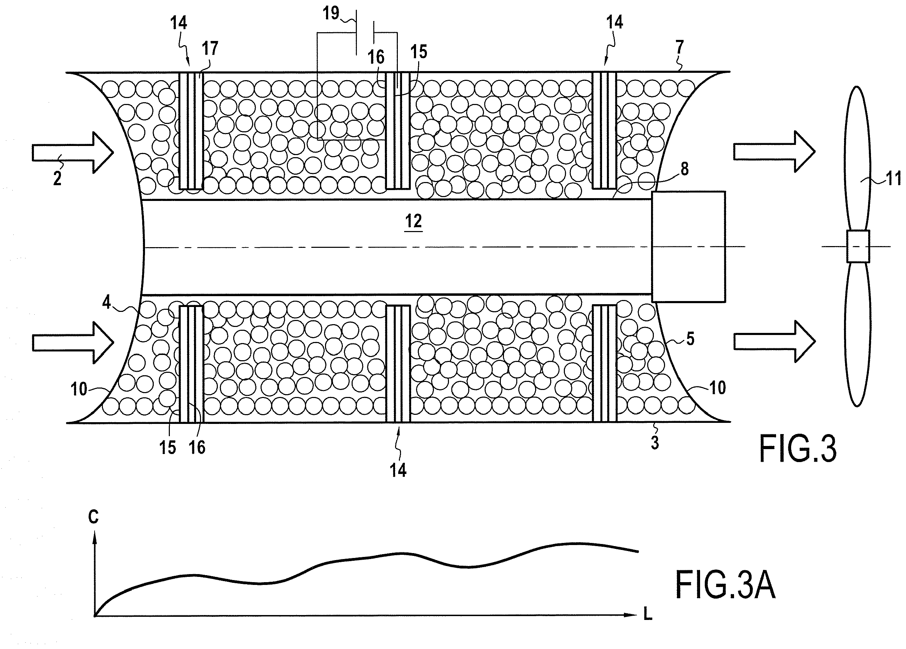

La publication de BAN J Y ET AL « Highly concentrated toluene decomposition on the dielectric barrier discharge (DBD) plasma-photocatalytic hybrid system with Mn-Ti-incorporated mesoporous silicate photocatalyst (MN-Ti-MPS) », Applied Surface Science, ELSEVIER, AMSTERDAM, NL, vol. 253, n° 2, 15 novembre 2006 (2006-11-15), pages 535-542, SP024893621, ISSN : 0169-4332, DOI : 10.1016/J.APSUSC.2005.12.103 [extrait le 2006-11-15] * chapitre 2.4 « Analysis of product for toluene décomposition » ; page 537 - page 538 ; figure 3» décrit une étude de l'utilisation d'un composé mésoporeux en silicate incorporant du Mn et du Ti pour la destruction du toluène dans un système hybride basé sur un traitement plasma et la photocatalyse. A cet effet, le réacteur décrit comporte un réacteur à plasma suivi en série par un réacteur photocatalytique contenant un agent photocatalyseur. La dégradation du toluène se fait par le passage du toluène d'abord dans le réacteur plasma qui, via la décharge, permet une pré-dégradation du toluène à l'aide de la formation d'espèces ioniques puis une minéralisation en CO2 des sous-produits du réacteur photocatalytique.

Cette technique s'applique exclusivement à la destruction du toluène, par la mise en oeuvre d'une pré-dégradation par ionisation suivie par une minéralisation, ce qui limite les applications d'une telle technique. Par ailleurs, cette technique ne permet pas d'obtenir un traitement performant car la technologie dégrade les polluants selon deux réactions successives :
- -des réactions d'addition et de substitution des espèces ioniques générés formant des produits intermédiaires secondaires de réactions pouvant être plus toxiques que les polluants initiaux,
- puis des oxydations photocatalytiques de ces derniers dont la performance dépend de la capacité oxydante déterminée par le montage du réacteur photocatalytique ne permettant pas d'obtenir une minéralisation optimale du toluène.

Les produits intermédiaires secondaires formés à la suite du premier réacteur plasma entrent en compétition avec le toluène lors de leur dégradation par voie radicalaire dans le second réacteur photocatalytique. Il y a obligatoirement des espèces toxiques qui ne seront pas réduites comme cela apparait sur les figures 10 et 11 qui montrent les limites d'une telle technique en obtenant des conversions de 27 à 43,87 % en CO2 et l'apparition d'intermédiaires réactionnels secondaires toxiques dont le benzène (CMR).

Par ailleurs, il doit être constaté que les tests de minéralisation du toluène sont effectués avec une injection de 1000 ppm de toluène ne permettant pas une utilisation d'un tel système pour des applications industrielles nécessitant un traitement en continu des effluents gazeux.

La présente invention vise donc à remédier aux inconvénients de l'état de la technique en proposant une technique de traitement des effluents combinant simultanément la photocatalyse sous irradiation UV et la production de plasma froid, cette technique étant adaptée pour contrôler les espèces oxydantes produites afin d'améliorer les performances de destruction des composés organiques dans les effluents de toute origine.

La présente invention vise donc à proposer un procédé de traitement d'effluents circulant entre l'entrée et la sortie d'un réacteur, consistant à soumettre les effluents à un traitement au plasma froid à l'aide d'au moins un dispositif de décharge à barrière diélectrique et à l'action d'un agent photocatalyseur sous rayonnement UV en vue de la production d'espèces oxydantes pour le traitement des effluents.

Selon l'invention, le procédé consiste à réaliser le traitement au plasma froid de manière intégrée et localisée à l'intérieur d'un lit de microbilles poreuses porteuses de l'agent photocatalyseur et placées à l'intérieur du réacteur en entourant chaque dispositif de décharge à barrière diélectrique permettant la génération d'espèces oxydantes ainsi que leur diffusion à l'intérieur du lit.

De plus, le procédé selon l'invention peut présenter en outre en combinaison au moins l'une et/ou l'autre des caractéristiques additionnelles suivantes :
- réaliser un lit fluidisé de microbilles poreuses à l'intérieur du réacteur,
- soumettre les effluents successivement entre l'entrée et la sortie du réacteur, à au moins un cycle de traitement comportant un traitement photocatalytique et une absorption partielle par les microbilles poreuses, un traitement photocatalytique combiné à un traitement au plasma froid et une absorption partielle par les microbilles poreuses, un traitement photocatalytique et une absorption partielle par les microbilles poreuses.

Un autre objet de l'invention est de proposer un réacteur pour la mise en oeuvre du procédé de traitement.

Selon l'invention, le réacteur comprend :
- une enceinte délimitant un circuit de circulation pour les effluents entre une entrée et une sortie et à l'intérieur de laquelle est installé un lit de microbilles poreuses porteuses d'un agent photocatalyseur sous rayonnement UV,
- au moins une source d'irradiation UV des microbilles poreuses,
- au moins un dispositif de décharge à barrière diélectrique génératrice de plasma froid, intégré de manière localisée à l'intérieur du lit de microbilles poreuses pour être entouré par les microbilles poreuses et pour agir simultanément à l'irradiation UV, de manière à assurer la génération d'espèces oxydantes ainsi que leur diffusion à l'intérieur du lit.

De plus, le réacteur selon l'invention peut présenter en outre en combinaison au moins l'une et/ou l'autre des caractéristiques additionnelles suivantes :
- l'enceinte est délimitée par des parois externe et interne concentriques entre elles, la source d'irradiation UV étant montée dans le logement délimité par la paroi interne tandis qu'une série de dispositifs de décharge à barrière diélectrique sont intégrés à l'intérieur de l'enceinte,
- les dispositifs de décharge à barrière diélectrique sont disposés soit axialement en étant répartis angulairement dans l'enceinte soit radialement à partir de la paroi interne selon une ou plusieurs sections de passage de l'enceinte,
- l'enceinte est délimitée par un caisson allongé présentant sur l'une de ses faces l'entrée pour les effluents et sur la face opposée, la sortie pour les effluents qui est décalée latéralement par rapport à l'entrée, le caisson contenant le lit de microbilles poreuses dans lequel sont logés le dispositif de décharge à barrière diélectrique et la source d'irradiation UV,
- l'enceinte est délimitée par une paroi périphérique externe délimitant l'entrée et raccordée à deux parois transversales dont l'une délimite la sortie,
- l'enceinte comporte une série de modules de traitement disposés en chicane à l'intérieur de l'enceinte et comportant chacun un support orientable supportant des sources d'irradiation UV et des dispositifs de décharge,
- l'enceinte est réalisée sous la forme d'une cartouche démontable comportant un culot de montage pourvu de moyens de fixation temporaire et de bornes de connexion électrique pour le dispositif de décharge à barrière diélectrique et de la source d'irradiation UV, le lit de microbilles poreuses étant maintenu sur le culot à l'aide d'une grille de maintien perméable aux effluents à traiter,
- un système pour fluidiser le lit de microbilles poreuses,
- le système pour fluidiser le lit de microbilles poreuses assure une mise en surpression ou en dépression du lit de microbilles poreuses,
- les microbilles poreuses possèdent des tailles de pores comprises entre 3 et 10 Angströms,
- les microbilles poreuses possèdent un diamètre compris entre 500 µm et 5 cm et de préférence entre 1 000 µm et 8 000 µm,
- les microbilles poreuses sont en ilménite, zéolithe, Charbon actif et/ou en Permanganate de Potassium,
- l'agent photocatalyseur des microbilles poreuses est pris seul ou en combinaison parmi la liste suivante : ilménite, TiO2, ZnO, MO, Métaux lourds,
- la source d'irradiation UV émet dans une longueur d'onde comprise entre 150 et 420 nm,
- la source d'irradiation UV est formée par une série de diodes UV réparties à la périphérie de la grille de maintien.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est une vue en coupe longitudinale d'un premier exemple de réalisation d'un réacteur de traitement conforme à l'invention.
La **Figure 1A** est un schéma illustrant la variation de la capacité oxydante C obtenue à l'intérieur du réacteur selon le trajet **I** des effluents.
La **Figure 2** est une vue en coupe transversale prise sensiblement selon les lignes II-II de la **Fig. 1** du réacteur conforme à l'invention.
La **Figure 2A** est une vue montrant la variation de la capacité oxydante **C** obtenue à l'intérieur du réacteur selon une section droite transversale **S** dudit réacteur.
La **Figure 3** est une vue en coupe longitudinale d'un deuxième exemple de réalisation d'un réacteur conforme à l'invention.
La **Figure 3A** est une vue montrant l'évolution de la capacité oxydante **C** obtenue à l'intérieur du réacteur selon le trajet longitudinal **I** des effluents.
La **Figure 4** est une vue en coupe transversale prise sensiblement selon les lignes IV-IV de la **Fig. 3****.**
La **Figure 4A** est une vue montrant l'évolution de la capacité oxydante **C** obtenue à l'intérieur du réacteur selon une section transversale **S** du réacteur.
Les **Figures 5** à **9** illustrent diverses autres formes de réalisation de réacteurs conformes à l'invention.

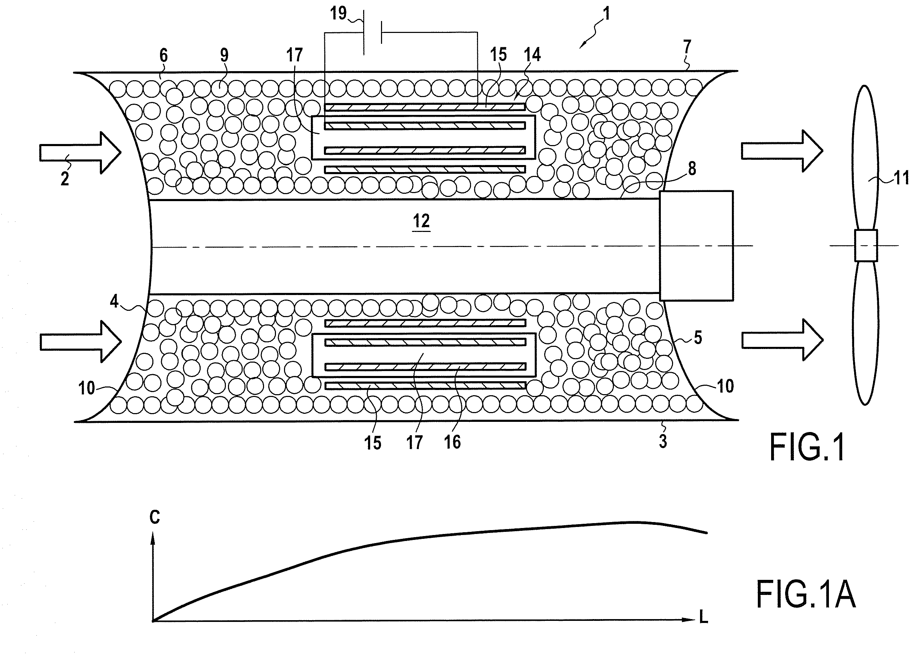

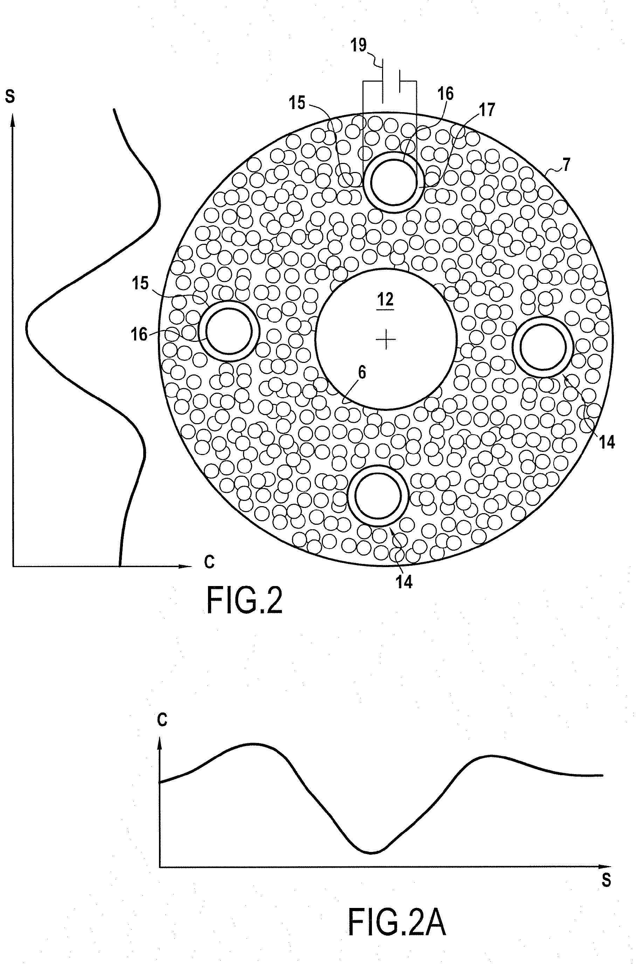

Les **Fig. 1** et **2** illustrent un premier exemple de réalisation d'un réacteur **1** conforme à l'invention pour traiter des effluents **2** au sens général. Les effluents **2** sont de nature gazeuse ou aqueuse comportant des polluants tels que par exemple des particules, des micro-organismes type virus, bactéries, moisissures et algues et les polluants chimiques type COV, SCOV, BTEX, HAP (C10-C25), HAAA, NOx, SOx, H2S, CO, 03, des composés halogénés, des perturbateurs endocriniens et l'ensemble des molécules olfactives.

Le réacteur **1** comporte un corps ou enceinte **3** présentant une entrée **4** pour les effluents et une sortie **5** pour les effluents traités. L'enceinte **3** délimite intérieurement une chambre **6** formant un circuit de circulation pour les effluents entre l'entrée **4** et la sortie **5,** amenés à circuler de manière discontinue ou continue.

Dans l'exemple de réalisation illustré, l'enceinte **3** comporte une paroi externe **7** de section circulaire à l'intérieur de laquelle est montée une paroi interne **8** de section également circulaire. Les deux parois interne **8** et externe **7** se présente sous la forme de deux parois tubulaires montées de manière concentrique entre elles. Ainsi, la chambre **6** présente une forme annulaire. Bien entendu, la forme du réacteur peut être différente d'une structure tubulaire de section circulaire.

Selon une caractéristique de l'invention, un lit de microbilles poreuses **9** est installé à l'intérieur de l'enceinte **3.** Comme cela sera expliqué dans la suite de la description, les microbilles poreuses **9** sont placées dans le circuit de circulation **6** de manière que les effluents traversent le lit de microbilles poreuses lors de leur progression entre l'entrée **4** et la sortie **5** du réacteur. Selon cet exemple de réalisation, l'enceinte **3** comporte deux parois transversales perméables aux effluents et permettant de maintenir en position les microbilles poreuses **9.** Ainsi par exemple, l'enceinte **3** comporte des grilles **10** en tant que système de maintien des microbilles **9**

Selon une variante de réalisation, il est à noter que le lit de microbilles poreuses peut être fluidisé. Selon cette variante de réalisation, le réacteur **1** comporte un système **11** pour fluidifier le lit de microbilles poreuses. Par exemple, le système **11** pour fluidifier le lit de microbilles poreuses **9** assure une mise en surpression ou en dépression du lit de microbilles poreuses. Par exemple, le système **11** peut être une pompe ou un ventilateur.

Les microbilles poreuses possèdent avantageusement, une taille de pores comprise entre 3 et 10 000 Angströms, avantageusement entre 3 et 5 000 Angströms et de préférence encore entre 3 et 10 Angströms.

Par exemple, chaque microbille poreuse **9** présente une forme sphérique et possède un diamètre compris entre 500 µm et 5 cm et de préférence entre 1 000 µm et 8 000 µm.

Les microbilles poreuses sont en ilménite, zéolithe, Charbon actif et/ou en Permanganate de Potassium.

Selon une autre caractéristique de l'invention, les microbilles poreuses **9** sont porteuses d'un agent photocatalyseur sous rayonnement UV.

L'agent photocatalyseur des microbilles poreuses **9** est pris seul ou en combinaison parmi la liste suivante : ilménite, TiO2, ZnO, MO, Métaux lourds.

Il doit être compris que les microbilles poreuses **9** sont réalisées en un ou plusieurs matériaux. Les microbilles poreuses **9** peuvent être réalisées en un seul matériau si ce dernier est un agent photocatalyseur. Ainsi, par exemple, les microbilles poreuses **9** peuvent être réalisées uniquement en ilménite.

Selon une variante de réalisation, toutes les microbilles poreuses **9** du lit placé à l'intérieur du réacteur **1** sont identiques. Selon une autre variante de réalisation, le réacteur **1** peut contenir des microbilles poreuses **9** différentes tant au niveau de leurs matériaux constitutifs que de leurs porosités et de leurs diamètres. Les microbilles poreuses **9** peuvent ainsi avoir des tailles de pores homogènes ou hétérogènes allant de préférence de 3 à 10 Angströms. Les microbilles poreuses **9** peuvent aussi posséder un diamètre homogène ou hétérogène allant de 500 µm à 5 cm. Selon une autre caractéristique de l'invention, le réacteur **1** comporte au moins une et dans l'exemple illustré aux **Fig. 1** à **4****,** une source d'irradiation UV **12** des microbilles poreuses **9.** Dans l'exemple illustré, la source d'irradiation UV **12** est montée dans le logement aménagé à l'intérieur de la paroi interne tubulaire **8.** La source d'irradiation UV **12** est montée de manière que l'émission UV puisse agir sur les agents photocatalyseurs des microbilles poreuses **9** installées dans l'enceinte **3.** La source d'irradiation UV émet dans une longueur d'onde comprise entre 150 et 420 nm et de préférence entre 180 et 365 nm. Bien entendu, dans le cas d'une enceinte **3** de plus grande taille ou de forme différente, le réacteur **1** peut être équipé de plusieurs sources d'irradiation UV **12** pour permettre d'irradier l'ensemble des microbilles poreuses **9** placées à l'intérieur de l'enceinte **3.**

Le réacteur **1** comporte également au moins un et dans l'exemple illustré aux **Fig. 1** et **2****,** quatre dispositifs **14** de décharge à barrière diélectrique génératrice de plasma froid, intégrés de manière localisée à l'intérieur du lit de microbilles poreuses **9.** Chaque dispositif de décharge à barrière diélectrique **14** comporte deux électrodes **15, 16** séparées par un diélectrique **17.** Les électrodes **15, 16** sont reliées à une source de tension **19** continue ou alternative de 12 à 220 Volts. Il doit être noté que chaque dispositif de décharge à barrière diélectrique **14** est plongé dans le lit de microbilles poreuses **9** de sorte que chaque dispositif de décharge à barrière diélectrique **14** est entouré par des microbilles poreuses **9.** Il doit être noté que les microbilles poreuses **9** ne peuvent pas se positionner entre les électrodes **15, 16** mais sont situées à l'extérieur des électrodes **15, 16.**

Dans l'exemple illustré aux **Fig. 1** et **2****,** les dispositifs de décharge à barrière diélectrique **14** sont disposés axialement en étant répartis angulairement dans l'enceinte **3.** Chaque dispositif de décharge à barrière diélectrique **14** est décalé de 90° par rapport au dispositif voisin, en s'étendant sur une longueur limitée inférieure à la longueur axiale du réacteur. Tel que cela ressort des **Fig. 1** et **2****,** chaque dispositif de décharge à barrière diélectrique **14** s'étend ainsi à distance par rapport aux parois internes **8** et externe **7,** et à distance des extrémités du réacteur **1** de manière à pouvoir être totalement intégré ou entouré par les microbilles poreuses **9.**

Le réacteur **1** décrit ci-dessus conforme à l'invention permet de mettre en oeuvre un procédé de traitement particulièrement efficace.

En effet, le procédé selon l'invention vise à soumettre les effluents simultanément à un traitement au plasma froid (via le ou les dispositifs de décharge **14**) et à l'action d'un agent photocatalyseur sous rayonnement UV (via la source d'irradiation UV **12** agissant sur les microbilles poreuses **9**) permettant la génération optimale d'espèces actives oxydantes composées de manière avantageuse, majoritairement de radicaux formant un nuage radicalaire favorisant le traitement et la minéralisation optimale des effluents consiste à réaliser le traitement au plasma froid de manière intégrée et localisée à l'intérieur du lit de microbilles poreuses **9** placées à l'intérieur du réacteur **1** et porteuses de l'agent photocatalyseur, de sorte que les microbilles poreuses **9** ne subissent pas de décharge électrique via la barrière diélectrique des dispositifs de décharge à barrière diélectrique **14.** Un tel procédé permet de générer des espèces actives ou oxydantes, de nature différente majoritairement des ions et de l'ozone pour le plasma et des radicaux hydroxydes et oxygènes radicalaires pour la photocatalyse. Ces composés ou espèces actives ou oxydantes générés séparément mais simultanément par le plasma froid et les microbilles poreuses **9** autocatalytiques irradiées par la source UV **12,** permettent la destruction et la minéralisation des composés chimiques, des micro-organismes ainsi que la rétention des particules et la régénération des microbilles poreuses **9.** Un tel principe permet de gérer de manière pertinente des temps de vie et cinétiques réactionnels différents au sein du réacteur **1** lors de la génération des espèces actives ou oxydantes. Les temps de vie et cinétiques réactionnelles sont comprises, selon la nature de l'espèce oxydante ou réactive (ions ou radicaux) (et / ou du mélange d'espèces réactives composant la capacité oxydante du réacteur) et celles des polluants, entre 10⁻⁹ seconde et quelques secondes.

Le procédé selon l'invention permet ainsi que le taux des espèces oxydantes produites à l'intérieur du lit évolue entre l'entrée **4** et la sortie **5** du réacteur **1** assurant ainsi la génération locale d'espèces oxydantes, ainsi que leur diffusion à l'intérieur du lit. L'obtention d'un tel gradient de production de l'espèce oxydante au sein de l'enceinte **3** permet ainsi la dégradation simultanée de mélange complexe de polluants chimiques, de contaminants micro-organiques et particules, aboutissant à une minéralisation de ces derniers en CO2, H2O, N2, O2 et H2O2. Tel que cela ressort de la **Fig. 1A****,** le taux des espèces oxydantes produites à l'intérieur du lit de microbilles poreuses ou la capacité oxydante **C** du réacteur progresse ou augmente entre l'entrée **4** et la sortie **5** du réacteur.

Le réacteur **1** selon l'invention permet ainsi de favoriser la formation d'espèces radicalaires et de H2O2 par effet fenton. La formation de H2O2 permet d'augmenter la capacité oxydante **C** interne du réacteur et permet d'augmenter l'effet germicide total obtenu. L'H2O2 rémanent est localement consommé lors de l'attaque de germes et/ou la formation de OH. Il doit être compris que le procédé selon l'invention vise ainsi à soumettre les effluents successivement entre l'entrée et la sortie du réacteur, à au moins un cycle de traitement comportant :
- un traitement photocatalytique et une absorption partielle par les microbilles poreuses **9** dans la zone située entre l'entrée **4** et les dispositifs de décharge à barrière diélectrique **14,**
- un traitement photocatalytique combiné à un traitement à plasma froid et une absorption partielle par les microbilles poreuses **9** au niveau de la chambre du réacteur **1** dans laquelle sont placés les dispositifs de décharge à barrière diélectrique **14,**
- un traitement photocatalytique et une absorption partielle par les microbilles poreuses **9** dans la zone située entre la sortie **5** du réacteur et les dispositifs de décharge à barrière diélectrique **14.**

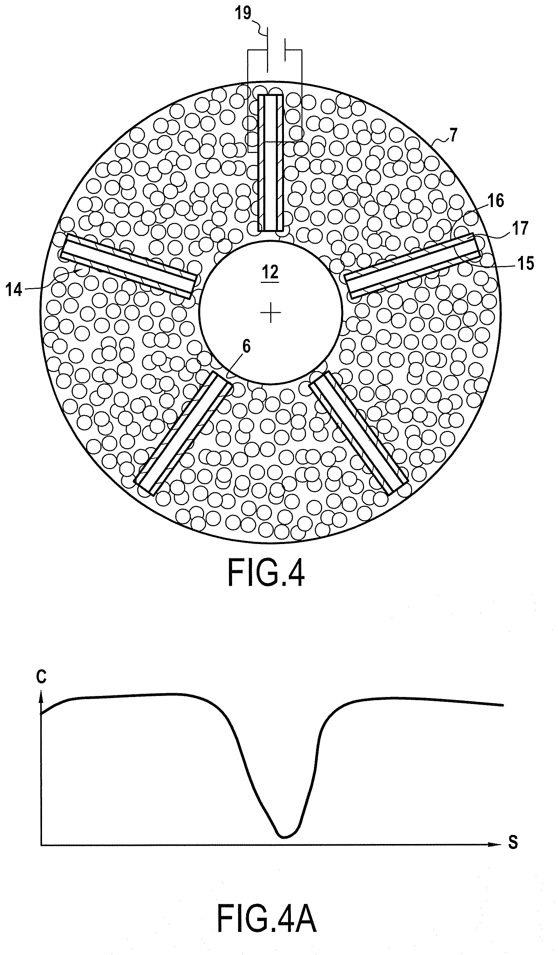

Les **Fig. 3** et **4** illustrent un autre exemple de réalisation du réacteur **1** selon l'invention dans lequel le réacteur **1** comporte des dispositifs de décharge à barrière diélectrique **14** répartis radialement dans l'enceinte. Selon cet exemple, les dispositifs de décharge à barrière diélectrique **14** sont répartis radialement selon plusieurs sections de passage de l'enceinte **3.** Chaque section de passage de l'enceinte **3** comporte cinq dispositifs de décharge à barrière diélectrique **14** s'étendant radialement à partir de la paroi interne **6** du réacteur **1.** Dans l'exemple illustré aux **Fig. 3** et **4****,** le réacteur **1** comporte trois séries de dispositifs de décharge à barrière diélectrique **14** répartis entre l'entrée **4** et la sortie **5** du réacteur **1.** Le principe de fonctionnement du réacteur **1** illustré aux **Fig. 3** à **4** est analogue au principe de fonctionnement du réacteur **1** illustré aux **Fig. 1** et **2****.** Ainsi, le taux des espèces oxydantes produites à l'intérieur du lit de microbilles poreuses ou la capacité oxydante **C** du réacteur progresse ou augmente entre l'entrée **4** et la sortie **5** du réacteur **1** (**Fig. 3A**). Toutefois, le taux des espèces oxydantes produites connaît un tassement voire une légère diminution entre deux séries voisines de dispositifs de décharge à barrière diélectrique **14.** Le positionnement en série des dispositifs de décharge à barrière diélectrique **14** le long du trajet des effluents permet d'augmenter globalement la capacité oxydante du réacteur comme cela apparaît clairement à la **Fig. 3A****,** entre l'entrée et la sortie du réacteur.

Bien entendu, il peut être prévu d'équiper le réacteur **1** selon une seule section, de dispositifs de décharge à barrière diélectrique **14** s'étendant radialement. De même, il peut être prévu de combiner dans un même réacteur **1,** des dispositifs de décharge à barrière diélectrique **14** s'étendant soit axialement, soit radialement.

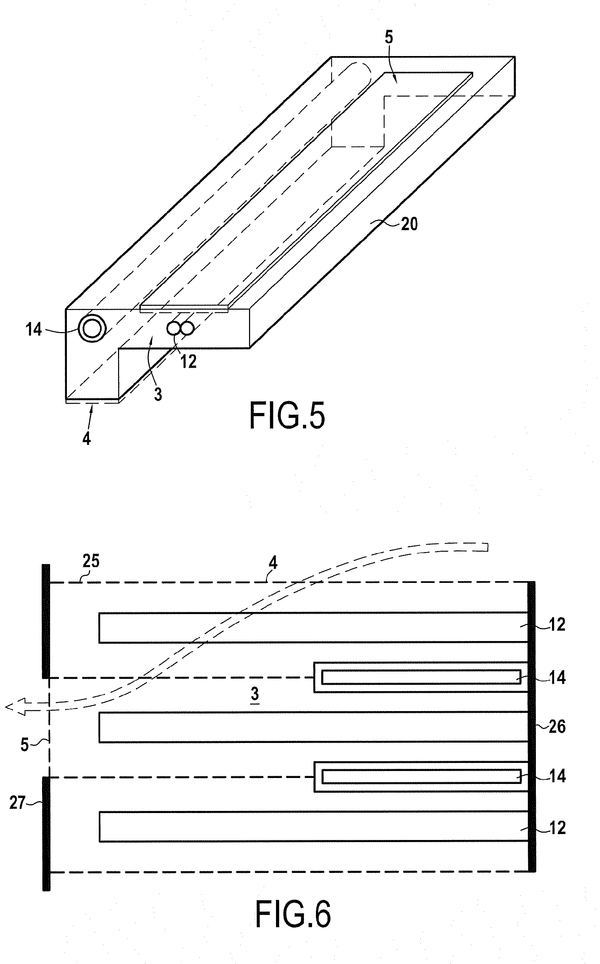

La **Fig. 5** illustre un autre exemple de réalisation d'un réacteur conforme à l'invention se présentant sous la forme d'un caisson allongé **20** délimitant intérieurement l'enceinte **3.** Le caisson **20** présente sur l'une de ses faces principales l'entrée **4** pour les effluents et sur sa face principale opposée la sortie **5** pour les effluents. L'entrée **4** et la sortie **5** s'étendent sur tout ou partie de la longueur du caisson allongé **20,** en étant décalées latéralement l'une par rapport à l'autre pour permettre aux effluents de circuler à l'intérieur des microbilles poreuses **9** montées à l'intérieur de l'enceinte **3** et à l'intérieur de laquelle sont montées une ou plusieurs sources d'irradiation UV **12** s'étendant longitudinalement à l'intérieur du caisson **20** et un ou plusieurs dispositifs de décharge **14** s'étendant également longitudinalement à l'intérieur du caisson **20.** Par exemple, la source d'irradiation UV **12** est disposée en face de la sortie **5** tandis que le dispositif de décharge **14** est situé en face de l'entrée **4.**

La **Fig. 6** illustre une autre variante de réalisation d'un réacteur **1** dans lequel l'enceinte **3** est délimitée par une paroi périphérique externe **25** délimitant l'entrée pour les effluents à traiter. Cette paroi périphérique **25** est raccordée à deux parois transversales **26,27** dont l'une par exemple **27** délimite la sortie **5** des effluents. L'enceinte **3** comporte une ou plusieurs sources d'irradiation UV **12** s'étendant axialement à l'intérieur de l'enceinte **3** à partir de la paroi **26** opposée de celle pourvue de la sortie **5.** Le réacteur comporte également un ou plusieurs dispositifs de décharge **14** s'étendant axialement à partir de la paroi **26** voire également de la paroi **25** pourvue de la sortie **5.** Les effluents entrent ainsi tangentiellement dans l'enceinte **3** et en ressortent axialement par la sortie **5.**

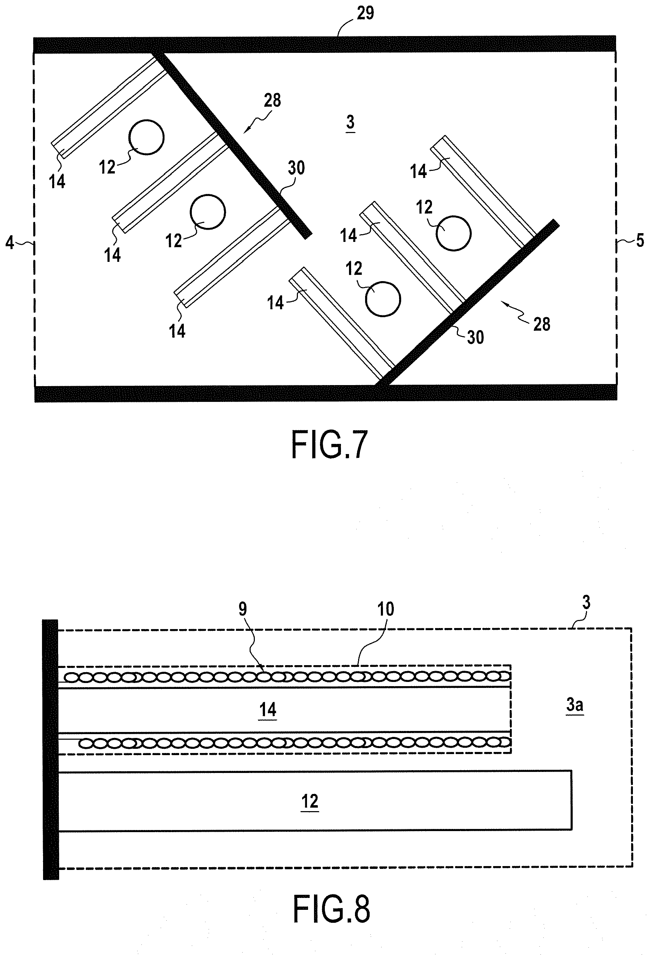

La **Fig. 7** illustre une autre variante de réalisation du réacteur **1** comportant une série de modules de traitements **28** disposés en chicane à l'intérieur de l'enceinte **3** délimitée par un caisson **29.** Chaque module de traitement **28** comporte un support **30** équipé d'une ou de plusieurs sources d'irradiation UV **12** et d'un ou de plusieurs dispositifs de décharge **14.** Les modules de traitement **28** sont placés successivement sur le trajet des effluents entre l'entrée **4** et la sortie **5** aménagées selon deux parois transversales opposées du caisson **19.** Ainsi, chaque support **30** s'étend sur toute la largeur du caisson **29** avec les sources d'irradiation **UV 12** qui s'étendent selon la largeur du caisson alors que des dispositifs de décharges **14** s'élèvent à partir du support **30** en étant distribués de manière appropriée sur ce support **30.**

Une telle disposition des modules de traitement **28** à l'intérieur de l'enceinte **3** permet de créer un flux turbulent et de maîtriser les flux et les pertes de charges. Avantageusement, chaque support **29** est monté pour être orientable à volonté à l'intérieur du réacteur **1.**

Bien entendu, l'enceinte **3** des réacteurs illustrés aux **Fig. 5** à **7** comporte des microbilles poreuses **9** comme expliqué en relation des **Fig. 1** à **4****.** De telles microbilles poreuses **9** sont maintenues en position à l'intérieur de l'enceinte à l'aide d'un système de maintien **10** perméable aux effluents telle une grille de maintien.

Il est à noter que la totalité de l'enceinte **3** peut être remplie par les microbilles poreuses **9.** Cependant, il peut être envisagé que les microbilles poreuses **9** se trouvent confinées par un système de maintien **10** adapté pour entourer uniquement chaque dispositif de décharge **14.** Ainsi tel que cela ressort clairement de la **Fig. 8****,** une partie **3a** de l'enceinte **3** ne comporte pas de microbilles poreuses **9.** Bien entendu, une telle disposition peut être choisie pour toutes les variantes de réalisation de réacteur conforme à l'invention.

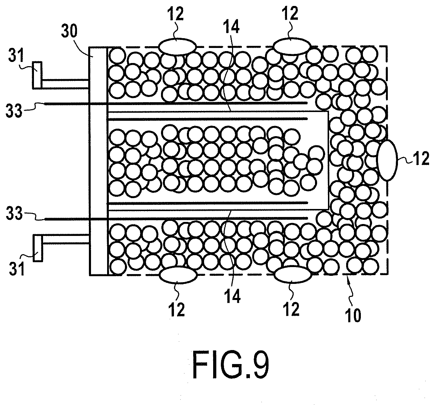

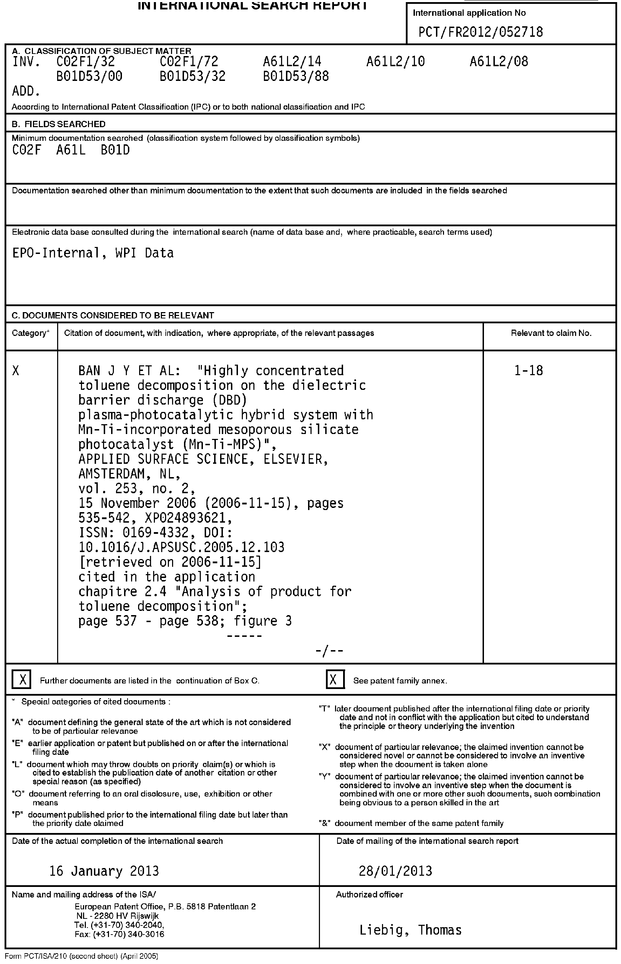

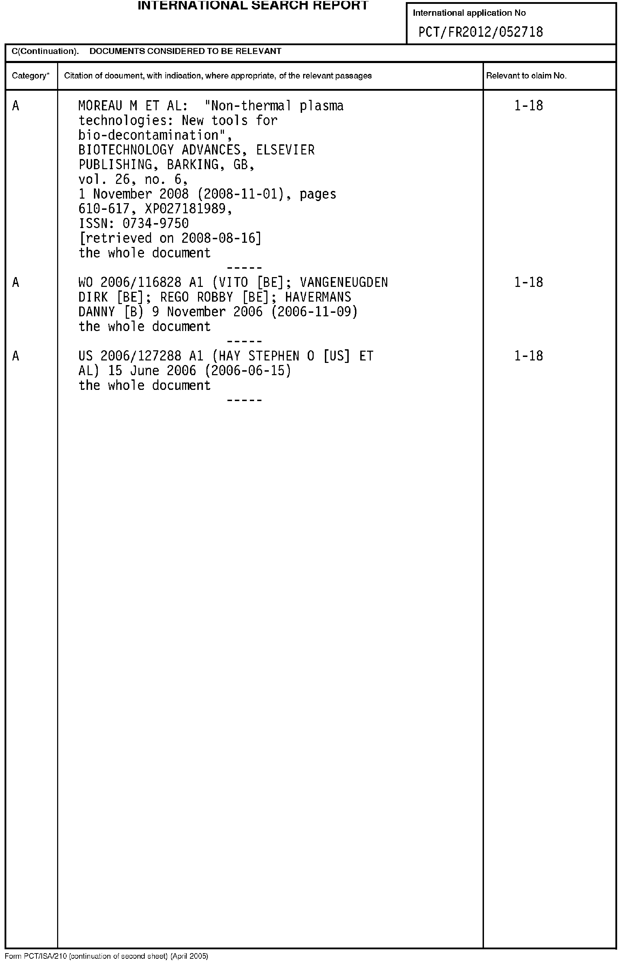

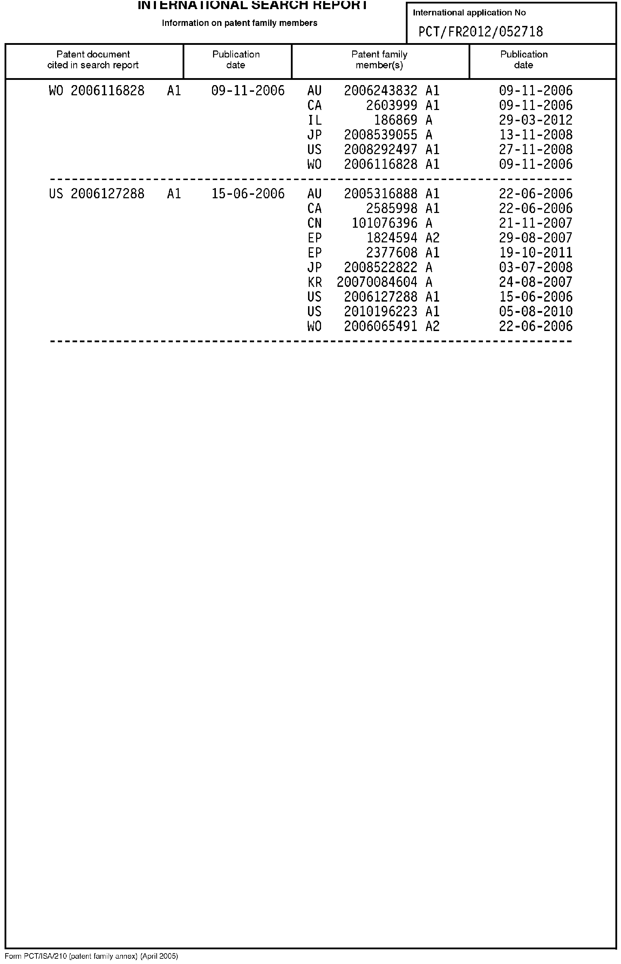

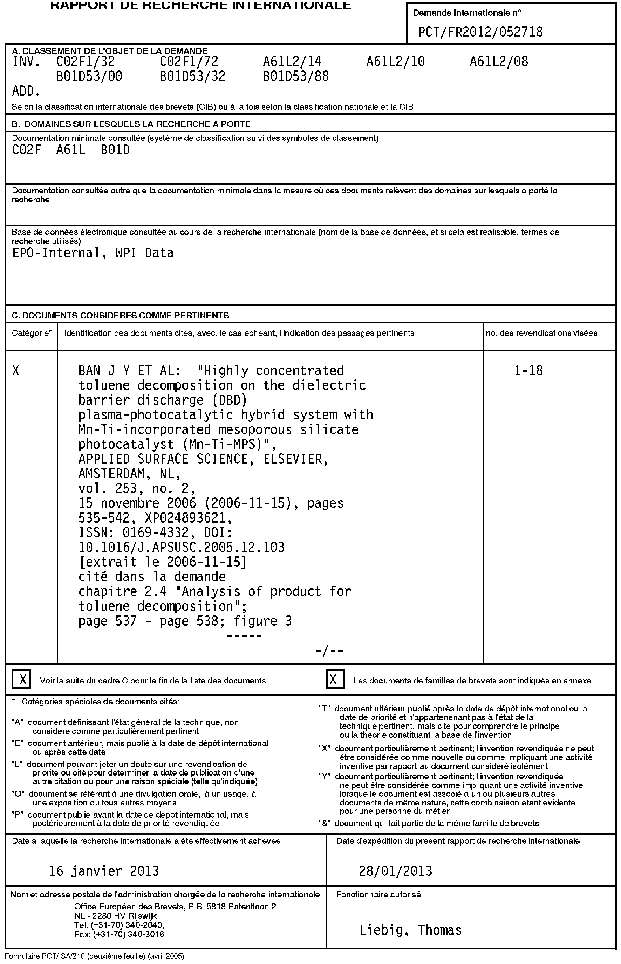

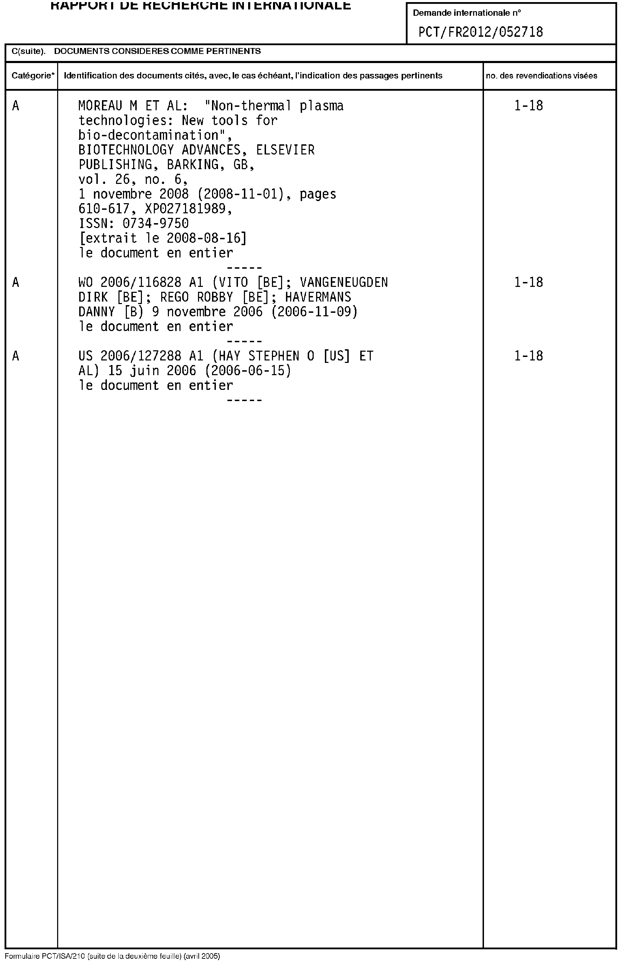

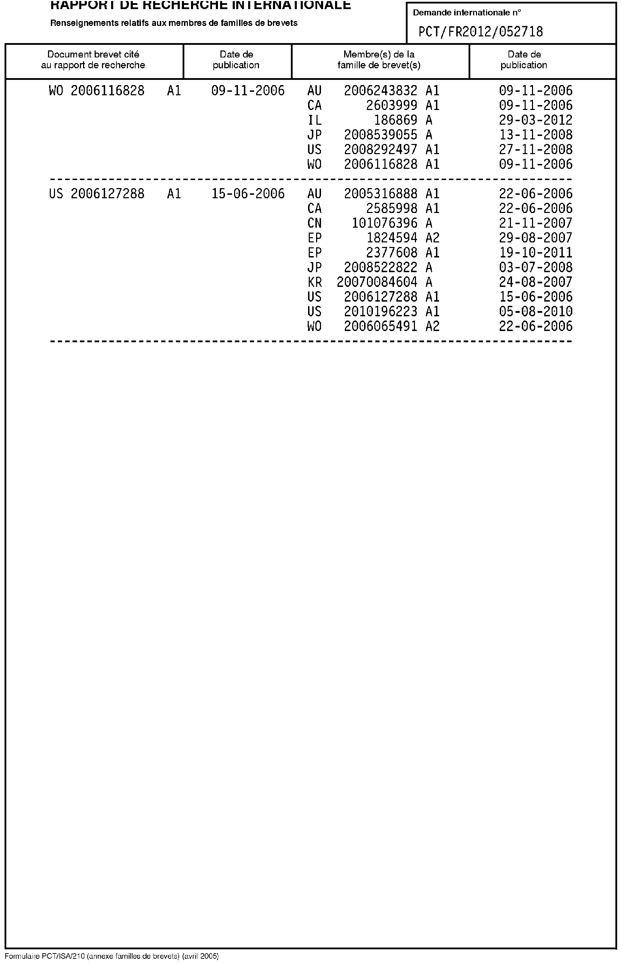

La **Fig. 9** illustre une autre variante de réalisation du réacteur **1** selon une configuration compacte et de préférence démontable. Le réacteur **1** est réalisé sous la forme d'une cartouche démontable comportant un culot de montage **30** pourvu de moyens de fixation temporaire **31** de tous types tels qu'à baïonnette ou à vis. Le culot **30** comporte également des bornes de connexion électriques **33** pour le dispositif de décharge **14** et la source de radiation UV **12.** Avantageusement, le dispositif de décharge **14** est monté sur le culot **30** en étant entouré par les microbilles poreuses **9** maintenues sur le culot **30** à l'aide d'une grille de maintien **10** perméable aux effluents à traiter. Selon une variante préférée de réalisation, mais non exclusivement, la source de radiation UV **12** est formée par une série de diodes UV réparties à la périphérie de la grille de maintien **10.**

Bien entendu, toutes les variantes de réalisation du réacteur selon l'invention comportent ou non un système **11** pour fluidiser le lit de microbilles poreuses **9.**

L'invention n'est pas limitée aux exemples décrits et représentés car diverses modifications peuvent y être apportées sans sortir de son cadre.

## Revendications

1. Procédé de traitement d'effluents (**2**) circulant entre l'entrée (**4**) et la sortie (**5**) d'un réacteur (**1**), consistant à soumettre les effluents à un traitement au plasma froid à l'aide d'au moins un dispositif de décharge à barrière diélectrique (**14**) et à l'action d'un agent photocatalyseur sous rayonnement UV en vue de la production d'espèces oxydantes pour le traitement des effluents, **caractérisé en ce qu'**il consiste à soumettre les effluents simultanément à un traitement au plasma froid et à l'action d'un agent photocatalyseur sous rayonnement UV, le traitement au plasma froid étant réalisé de manière intégrée et localisée à l'intérieur d'un lit de microbilles poreuses (**9**) porteuses de l'agent photocatalyseur et placées à l'intérieur du réacteur (**1**) en entourant chaque dispositif de décharge à barrière diélectrique (**14**) permettant d'assurer la génération d'espèces oxydantes, ainsi que leur diffusion à l'intérieur du lit.

2. Procédé de traitement selon la revendication 1, **caractérisé en ce qu'**il consiste à réaliser un lit fluidisé de microbilles poreuses (**9**) à l'intérieur du réacteur (**1**).

3. Procédé de traitement selon les revendications 1 ou 2, **caractérisé en ce qu'**il consiste à soumettre les effluents successivement entre l'entrée (**4**) et la sortie (**5**) du réacteur (**1**)**,** à au moins un cycle de traitement comportant un traitement photocatalytique et une absorption partielle par les microbilles poreuses (**9**), un traitement photocatalytique combiné à un traitement au plasma froid et une absorption partielle par les microbilles poreuses (**9**), un traitement photocatalytique et une absorption partielle par les microbilles poreuses (**9**).

4. Réacteur pour la mise en oeuvre du procédé de traitement conforme à l'une des revendications 1 à 3, **caractérisé en ce qu'**il comprend :
- une enceinte (**3**) délimitant un circuit (**6**) de circulation pour les effluents (**2**) entre une entrée (**4**) et une sortie (**5**) et à l'intérieur de laquelle est installé un lit de microbilles poreuses (**9**) porteuses d'un agent photocatalyseur sous rayonnement UV,
- au moins une source d'irradiation UV (**12**) des microbilles poreuses (**9**),
- au moins un dispositif (**14**) de décharge à barrière diélectrique génératrice de plasma froid, intégré de manière localisée à l'intérieur du lit de microbilles poreuses pour être entouré par les microbilles poreuses et pour agir simultanément à l'irradiation UV, de manière à assurer la génération d'espèces oxydantes et leur diffusion à l'intérieur du lit.

5. Réacteur selon la revendication 4, **caractérisé en ce que** l'enceinte (**3**) est délimitée par des parois externe et interne concentriques entre elles, une source d'irradiation UV (**12**) étant montée dans le logement délimité par la paroi interne (**8**) tandis qu'une série de dispositifs de décharge à barrière diélectrique (**14**) sont intégrés à l'intérieur de l'enceinte (**3**), l'enceinte (**3**) présentant des parois transversales délimitant respectivement l'entrée (**4**) et la sortie (**5**).

6. Réacteur selon la revendication 5, **caractérisé en ce que** les dispositifs de décharge à barrière diélectrique (**14**) sont disposés soit axialement en étant répartis angulairement dans l'enceinte (**3**) soit radialement à partir de la paroi interne (**8**) selon une ou plusieurs sections de passage de l'enceinte (**3**).

7. Réacteur selon la revendication 5, **caractérisé en ce que** l'enceinte (**3**) est délimitée par un caisson allongé (**20**) présentant sur l'une de ses faces l'entrée (**4**) pour les effluents et sur la face opposée, la sortie (**5**) pour les effluents qui est décalée latéralement par rapport à l'entrée, le caisson contenant le lit de microbilles poreuses (**9**) dans lequel sont logés le dispositif de décharge à barrière diélectrique (**14**) et la source d'irradiation UV (**12**).

8. Réacteur selon la revendication 5, **caractérisé en ce que** l'enceinte (**3**) est délimitée par une paroi périphérique externe délimitant l'entrée et raccordée à deux parois transversales dont l'une délimite la sortie (**5**).

9. Réacteur selon la revendication 5, **caractérisé en ce que** l'enceinte (**3**) comporte une série de modules de traitement disposés en chicane à l'intérieur de l'enceinte et comportant chacun un support orientable (**29**) supportant des sources d'irradiation UV (**12**) et des dispositifs de décharge (**14**).

10. Réacteur selon la revendication 5, **caractérisé en ce que** l'enceinte (**3**) est réalisée sous la forme d'une cartouche démontable comportant un culot de montage (**30**) pourvu de moyens de fixation temporaire (**31**) et de bornes de connexion électrique (**33**) pour le dispositif de décharge à barrière diélectrique (**14**) et de la source d'irradiation UV (**12**), le lit de microbilles poreuses (**9**) étant maintenu sur le culot à l'aide d'une grille de maintien (**10**) perméable aux effluents à traiter.

11. Réacteur selon l'une des revendications 4 à 10, **caractérisé en ce qu'**il comporte un système (**11**) pour fluidiser le lit de microbilles poreuses (**9**).

12. Réacteur selon la revendication 11, **caractérisé en ce que** le système (**11**) pour fluidiser le lit de microbilles poreuses (**9**) assure une mise en surpression ou en dépression du lit de microbilles poreuses.

13. Réacteur selon l'une des revendications 4 à 12, **caractérisé en ce que** les microbilles poreuses (**9**) possèdent des tailles de pores comprise entre 3 et 10 Angströms.

14. Réacteur selon l'une des revendications 4 à 13, **caractérisé en ce que** les microbilles poreuses (**9**) possèdent un diamètre compris entre 500 µm et 5 cm et de préférence entre 1 000 µm et 8 000 µm.

15. Réacteur selon l'une des revendications 4 à 14, **caractérisé en ce que** les microbilles poreuses (**9**) sont en ilménite, zéolithe, Charbon actif et/ou en Permanganate de Potassium.

16. Réacteur selon l'une des revendications 4 à 15, **caractérisé en ce que** l'agent photocatalyseur des microbilles poreuses (**9**) est pris seul ou en combinaison parmi la liste suivante : ilménite, TiO2, ZnO, MO, Métaux lourds.

17. Réacteur selon l'une des revendications 4 à 16, **caractérisé en ce que** la source d'irradiation UV (**12**) émet dans une longueur d'onde comprise entre 150 et 420 nm.

18. Réacteur selon la revendication 17, **caractérisé en ce que** la source d'irradiation UV (**12**) est formée par une série de diodes UV réparties à la périphérie de la grille de maintien (**35**).

## Patentansprüche

1. Verfahren zur Behandlung von Abgasen (2), die zwischen dem Einlass (4) und dem Auslass (5) eines Reaktors (1) zirkulieren, das darin besteht, die Abgase einer Behandlung mit Kaltplasma mittels mindestens einer dielektrischen Barriereentladungsvorrichtung (14) und der Wirkung eines Mittels zur Fotokatalyse unter UV-Strahlung zu unterziehen, um oxidierende Spezies für die Behandlung der Abgase herzustellen, **dadurch gekennzeichnet, dass** es darin besteht, die Abgase gleichzeitig einer Kaltplasmabehandlung und der Wirkung eines Mittels zur Fotokatalyse unter UV-Strahlung zu unterziehen, wobei die Kaltplasmabehandlung integriert und lokalisiert in einem Bett aus porösen Mikrokugeln (9) erfolgt, die das Fotokatalysemittel aufweisen und innerhalb des Reaktors (1) angeordnet sind und jede dielektrische Barriereentladungsvorrichtung (14) umgeben, wodurch die Erzeugung von oxidierenden Spezies sowie deren Diffusion innerhalb des Bettes sichergestellt werden kann.

2. Behandlungsverfahren gemäß Anspruch 1, **dadurch gekennzeichnet, dass** es darin besteht, ein Wirbelbett aus porösen Mikrokugeln (9) im Inneren des Reaktors (1) herzustellen.

3. Behandlungsverfahren gemäß den Ansprüchen 1 oder 2, **dadurch gekennzeichnet, dass** es darin besteht, die Abgase nacheinander zwischen dem Einlass (4) und dem Auslass (5) des Reaktors (1) mindestens einem Behandlungszyklus zu unterziehen, der eine fotokatalytische Behandlung und eine teilweise Absorption durch die porösen Mikrokugeln (9), eine fotokatalytische Behandlung kombiniert mit einer Behandlung mit Kaltplasma und eine teilweise Absorption durch die porösen Mikrokugeln (9), eine fotokatalytische Behandlung sowie eine teilweise Absorption durch die porösen Mikrokugeln (9) umfasst.

4. Reaktor zur Durchführung des Behandlungsverfahrens gemäß einem der Ansprüche 1 bis 3, **dadurch gekennzeichnet, dass** er umfasst:
- ein Gehäuse (3), das einen Zirkulationskreislauf (6) für die Abgase (2) zwischen einem Einlass (4) und einem Auslass (5) begrenzt und in dessen Innerem ein Bett aus porösen Mikrokugeln (9) eingerichtet ist, die ein Mittel zur Fotokatalyse unter UV-Strahlung tragen,
- mindestens eine UV-Strahlungsquelle (12) für die porösen Mikrokugeln (9),
- mindestens eine dielektrische Barriereentladungsvorrichtung (14), die ein Kaltplasma erzeugt und lokalisiert im Inneren des Bettes aus porösen Mikrokugeln integriert ist, um von den porösen Mikrokugeln umgeben zu werden und gleichzeitig mit der UV-Bestrahlung zu wirken, um die Erzeugung von oxidierenden Spezies und deren Diffusion innerhalb des Bettes sicherzustellen.

5. Reaktor gemäß Anspruch 4, **dadurch gekennzeichnet, dass** das Gehäuse (3) durch konzentrische Außen- und Innenwände dazwischen begrenzt ist, wobei in dem von der Innenwand (8) begrenzten Raum eine UV-Strahlungsquelle (12) angebracht ist, während eine Reihe von dielektrischen Barriereentladungsvorrichtungen (14) innerhalb des Gehäuses (3) integriert ist, wobei das Gehäuse (3) Querwände aufweist, die jeweils den Einlass (4) und den Auslass (5) begrenzen.

6. Reaktor gemäß Anspruch 5, **dadurch gekennzeichnet, dass** die dielektrischen Barriereentladungsvorrichtungen (14) entweder axial winkelverteilt im Gehäuse (3) oder radial von der Innenwand (8) in einem oder mehreren Durchgangsabschnitten des Gehäuses (3) angeordnet sind.

7. Reaktor gemäß Anspruch 5, **dadurch gekennzeichnet, dass** das Gehäuse (3) durch einen länglichen Kasten (20) begrenzt ist, der auf einer Seite den Einlass (4) für die Abgase und auf der gegenüberliegenden Seite den Auslass (5) für die Abgase aufweist, der seitlich gegenüber dem Einlass versetzt ist, wobei der Kasten das Bett aus porösen Mikrokugeln (9) enthält, in dem die dielektrische Barriereentladungsvorrichtung (14) und die UV-Strahlungsquelle (12) untergebracht sind.

8. Reaktor gemäß Anspruch 5, **dadurch gekennzeichnet, dass** das Gehäuse (3) durch eine den Einlass begrenzende äußere Umfangswand begrenzt und mit zwei Querwänden verbunden ist, von denen eine den Auslass (5) begrenzt.

9. Reaktor gemäß Anspruch 5, **dadurch gekennzeichnet, dass** das Gehäuse (3) eine Reihe von Behandlungsmodulen umfasst, die im Inneren des Gehäuses auf Lücke angeordnet sind und jeweils einen lenkbaren Träger (29) umfassen, der UV-Strahlungsquellen (12) und Entladungsvorrichtungen (14) trägt.

10. Reaktor gemäß Anspruch 5, **dadurch gekennzeichnet, dass** das Gehäuse (3) als herausnehmbare Kartusche ausgebildet ist, die einen mit temporären Befestigungsmitteln (31) und elektrischen Anschlussklemmen (33) für die dielektrische Barriereentladungsvorrichtung (14) und die UV-Strahlungsquelle (12) versehenen Montageboden (30) aufweist, wobei das Bett aus porösen Mikrokugeln (9) mittels eines für das zu behandelnde Abgas durchlässigen Haltegitters (10) auf dem Boden gehalten wird.

11. Reaktor gemäß einem der Ansprüche 4 bis 10, **dadurch gekennzeichnet, dass** er ein System (11) zur Fluidisierung des Bettes aus porösen Mikrokugeln (9) umfasst.

12. Reaktor gemäß Anspruch 11, **dadurch gekennzeichnet, dass** das System (11) zur Fluidisierung des Bettes aus porösen Mikrokugeln (9) die Druckbeaufschlagung oder Druckentlastung des Bettes aus porösen Mikrokugeln sicherstellt.

13. Reaktor gemäß einem der Ansprüche 4 bis 12, **dadurch gekennzeichnet, dass** die porösen Mikrokugeln (9) Porengrößen zwischen 3 und 10 Angström aufweisen.

14. Reaktor gemäß einem der Ansprüche 4 bis 13, **dadurch gekennzeichnet, dass** die porösen Mikrokugeln (9) einen Durchmesser zwischen 500 µm und 5 cm und vorzugsweise zwischen 1000 µm und 8000 µm aufweisen.

15. Reaktor gemäß einem der Ansprüche 4 bis 14, **dadurch gekennzeichnet, dass** die porösen Mikrokugeln (9) aus Ilmenit, Zeolith, Aktivkohle und/oder Kaliumpermanganat bestehen.

16. Reaktor gemäß einem der Ansprüche 4 bis 15, **dadurch gekennzeichnet, dass** das Mittel zur Fotokatalyse der porösen Mikrokugeln (9) allein oder in Kombination aus der folgenden Liste entnommen wird: Ilmenit, TiO2, ZnO, MO, Schwermetalle.

17. Reaktor gemäß einem der Ansprüche 4 bis 16, **dadurch gekennzeichnet, dass** die UV-Strahlungsquelle (12) in einer Wellenlänge zwischen 150 und 420 nm emittiert.

18. Reaktor gemäß Anspruch 17, **dadurch gekennzeichnet, dass** die UV-Strahlungsquelle (12) durch eine Reihe von UV-Dioden gebildet wird, die am Umfang des Haltegitters (35) verteilt sind.

## Claims

1. A process for treating effluents (2) moving between the inlet (4) and outlet (5) of a reactor (1), consisting in treating the effluents by means of a cold plasma treatment using at least one dielectric barrier discharge device (14) and by means of the action of a UV-photocatalytic agent in order to produce oxidizing species for treating the effluents, **characterized in that** it consists in treating the effluents with a cold plasma treatment simultaneously with the action of a UV-photocatalytic agent, the cold plasma treatment being carried out in a manner that is integrated into and located within a bed of porous microbeads (9) carrying the photocatalytic agent and placed inside the reactor (1) by surrounding each dielectric barrier discharge device (14) in order to generate oxidizing species as well as to diffuse them within the bed.

2. The treatment process according to claim 1, **characterized in that** it consists in producing a fluidized bed of porous microbeads (9) within the reactor (1) .

3. The treatment process according to claim 1 or claim 2, **characterized in that** it consists in carrying out at least one cycle of treatment on the effluents in succession between the inlet (4) and outlet (5) of the reactor (1), the treatment cycle comprising a photocatalytic treatment and partial absorption by the porous microbeads (9), a photocatalytic treatment combined with a cold plasma treatment and partial absorption by the porous microbeads (9), and a photocatalytic treatment and partial absorption by the porous microbeads (9).

4. A reactor for carrying out the treatment process according to any one of claims 1 to 3, **characterized in that** it comprises:
• a vessel (3) defining a circuit (6) for moving the effluents (2) between an inlet (4) and an outlet (5) and within which a bed of porous microbeads (9) carrying a UV-photocatalytic agent is installed;
• at least one source of UV irradiation (12) for the porous microbeads (9); and
• at least one dielectric barrier discharge device (14) generating cold plasma, integrated into the bed of porous microbeads in a localized manner and surrounded by porous microbeads and in order to carry out the simultaneous UV irradiation so as to generate oxidizing species as well as diffuse them within the bed.

5. The reactor according to claim 4, **characterized in that** the vessel (3) is defined by mutually concentric outer and inner walls, a source of UV irradiation (12) being mounted in the housing defined by the inner wall (8), while a series of dielectric barrier discharge devices (14) are integrated into the interior of the vessel (3), the vessel (3) having transverse walls respectively defining the inlet (4) and the outlet (5).

6. The reactor according to claim 5, **characterized in that** the dielectric barrier discharge devices (14) are disposed either axially, being distributed angularly in the vessel (3), or radially from the inner wall (8) in one or more cross sections of flow of the vessel (3).

7. The reactor according to claim 5, **characterized in that** the vessel (3) is defined by an elongate box (20) having the inlet (4) for the effluents on one of its faces and the outlet (5) for the effluents on the opposite face, which outlet is offset laterally relative to the inlet, the box containing the bed of porous microbeads (9) in which the dielectric barrier discharge device (14) and the source of UV irradiation (12) are accommodated.

8. The reactor according to claim 5, **characterized in that** the vessel (3) is defined by an outer peripheral wall defining the inlet and connected to two transverse walls, one of which defines the outlet (5).

9. The reactor according to claim 5, **characterized in that** the vessel (3) comprises a series of treatment modules in a staggered arrangement inside the vessel, each comprising a steerable support (29) supporting the sources of UV irradiation (12) and the dielectric barrier discharge devices (14).

10. The reactor according to claim 5, **characterized in that** the vessel (3) is produced in the form of a cartridge that can be detached, comprising a mounting base (30) provided with temporary fastener means (31) and electrical connection terminals (33) for the dielectric barrier discharge device (14) and the source of UV irradiation (12), the bed of porous microbeads (9) being retained on the base with the aid of a retaining screen (10) that is permeable to the effluents to be treated.

11. The reactor according to any one of claims 4 to 10, **characterized in that** it comprises a system (11) for fluidizing the bed of porous microbeads (9).

12. The reactor according to claim 11, **characterized in that** the system (11) for fluidizing the bed of porous microbeads (9) allows for over-pressurization or under-pressurization of the bed of porous microbeads.

13. The reactor according to any one of claims 4 to 12, **characterized in that** the porous microbeads (9) have pore sizes in the range 3 Å to 10 Å.

14. The reactor according to any one of claims 4 to 13, **characterized in that** the porous microbeads (9) have a diameter in the range 500 µm to 5 cm, preferably in the range 1000 µm to 8000 µm.

15. The reactor according to any one of claims 4 to 14, **characterized in that** the porous microbeads (9) are formed from ilmenite, zeolite, activated coal, and/or potassium permanganate.

16. The reactor according to any one of claims 4 to 15, **characterized in that** the photocatalytic agent of the porous microbeads (9) is taken from the following list, alone or in combination: ilmenite, TiO₂, ZnO, MO, and heavy metals.

17. The reactor according to any one of claims 4 to 16, **characterized in that** the source of UV irradiation (12) emits at a wavelength in the range 150 nm to 420 nm.

18. The reactor according to claim 17, **characterized in that** the source of UV irradiation (12) is formed by a series of UV diodes distributed at the periphery of the retaining screen (35).
